# FASCICULE DE BREVET EUROPEEN

(11) **EP 0 567 381 B1**
(45) Date de publication et mention de la délivrance du brevet: **26.06.1996**
(21) Numéro de dépôt: 93401002.6
(22) Date de dépôt: 16.04.1993
(51) Int. Cl.: C07C 17/25, C07C 21/06

(54) **Procédé de préparation du chlorure de vinyle par ultrapyrolyse du 1,2 dichloroethane**
Verfahren zur Herstellung von Vinylchlorid durch Ultrapyrolyse von 1,2-Dichlorethan
Process for the preparation of vinylchloride by ultrapyrolysis of 1,2-dichloroethane

(30) Priorité: 21.04.1992 FR 9204853
(43) Date de publication de la demande: 27.10.1993
(73) Titulaire: ELF ATOCHEM S.A., F-92800 Puteaux (FR)
(72) Inventeur: Le Blevec, Jean-Marc, F-56290 Port Louis (FR); Correia, Yves, F-04160 Chateau-Arnoux (FR); Masini, Jean-Jacques, F-69630 Chaponost (FR); Bousquet, Jacques, F-69540 Irigny (FR); Bergougnou, Maurice, London, Ontario N6G 1Z2 (CA)

(56) Documents cités:
- EP-A- 0 195 719
- FR-A- 1 132 804
- FR-A- 1 202 061
- CHEMICAL ABSTRACTS, vol. 97, no. 6, 1982, Columbus, Ohio, US; abstract no. 39512w, Y. UEMURA et al, "Vinyl chloride preparation", page 8, colonne 1; & BR-A-8 103 277

## Description

La présente invention concerne un procédé de préparation du chlorure de vinyle par ultrapyrolyse du 1,2 dichloroéthane, c'est à dire qu'on porte ce dernier à haute température le plus vite possible et après environ 0,1 à 0,5 secondes, on trempe pour bloquer toute réaction.

La pyrolyse du 1,2 dichloroéthane (D12) en phase gazeuse est le procédé industriel le plus utilisé pour produire le chlorure de vinyle, matière première du PVC. Ce procédé est décrit dans ULLMANN'S ENCYCLOPEDIA OF INDUSTRIAL CHEMISTRY 5^{e} édition, 1986, Volume A6, pages 287-289.

Selon ce procédé on chauffe le 1,2 dichloroéthane dans un four tubulaire côté tubes jusqu'à 500°C, le taux de conversion est de 50 à 60 % et la sélectivité 95 à 99 %, le temps de séjour est compris entre 10 et 20 secondes.

Si on veut augmenter la conversion on risque un dépôt de coke à l'intérieur des tubes. Bien que la demanderesse ait remédié à ces dépôts de coke par dilution du 1,2 dichloroéthane avec de l'acide chlorhydrique avant la pyrolyse comme le décrit le brevet européen EP 195 719, ce procédé de pyrolyse nécessite un appareillage très lourd et couteux.

Le brevet FR 1202061 décrit un procedé de préparation de chlorure de vinyl par pyrolyse du chlorure d'éthyle. Dans ce procedé, les durées de réaction sont tres courtes (0,1 à 0,01 secondes) et la formation de noir de fumée est supprimée par le fait que l'on effectue la pyrolyse à une température comprise entre 450°C et 900°C en amenant les calories par l'intermédiaire de vapeur d'eau surchauffée.

L'article de PAOUSCHKIN et CHARNAIA dans NEFTEKHIMIA vol 10, part 4, 1970, pages 583-585 décrit la pyrolyse du 1,2 dichloroéthane en présence de vapeur d'eau à raison de 2,5 parties d'eau pour 1 partie de 1,2 dichloroéthane. La pyrolyse se fait dans un réacteur dont les parois ont été couvertes par des dépôts carboniques, à une température entre 600 et 850°C. Il est écrit que "une fois traversé l'évaporateur à la température de 600°C la vapeur d'eau et le 1,2 dichloroéthane à une vitesse donnée ont été amenés dans le réacteur". Puis il est cité ensuite une vitesse volumétrique de 0,73 heure⁻¹ assurant un temps de contact égal à 0,003 secondes, ce qui est incohérent. On ne voit pas comment le mélange vapeur d'eau, 1,2 dichloroéthane peut traverser un évaporateur à 600°C, être chauffé entre 600 et 850°C, le tout en 0,003 secondes. De plus, les résultats montrent une forte sous production d'acétylène. Il est aussi très difficile d'opérer à l'échelle industrielle en présence d'eau, la séparation d'avec HCl étant difficile.

La demande de brevet européen EP 281 218 décrit un procédé de craquage d'hydrocarbures pour faire des oléfines dans lequel on met en contact pendant 10 à 100 millisecondes, (de préférence 20 à 50) un courant de particules préalablement chauffées entre 926°C et 1648°C avec un courant de naphta ou d'huiles lourdes préalablement chauffé entre 260°C et 690°C dans lequel on ajoute toujours de la vapeur d'eau (steam cracking). Le rapport en poids des particules au courant de naphta à craquer est de 5 à 200, c'est à dire que la plus basse température atteinte par le naphta est dans le cas des particules à 926°C, du naphta préchauffé à 260°C et le rapport particules/naphta valant 5 soit environ 800°C.

La plus haute température atteinte par le naphta est dans le cas des particules à 1648°C, du naphta préchauffé à 690°C et le rapport particules/naphta valant 200 soit environ 1648°C. En page 24, lignes 56-58 il est suggéré d'utiliser cette technique pour pyrolyser le 1,2 dichloroéthane c'est à dire qu'on suggère de le pyrolyser entre 800 et 1648°C de préférence pendant 0,02 à 0,05 secondes et en présence d'eau.

Si on utilise l'équation cinétique de D.H.R BARTON (JOURNAL OF CHEMICAL SOCIETY, 1949, page 148) on trouve qu'à 570°C pendant 0,160 s dans un réacteur de rapport surface volume 3,6 cm⁻¹ on obtient 2,4 % de conversion du 1,2 dichloroéthane, le 1,2 dichloroéthane ayant été préalablement dilué à l'azote, rapport molaire azote/1,2 dichloroéthane = 10.

Avec un temps de 0,050 s on obtient une conversion de 0 (zéro) %.

De plus on sait qu'en opérant à haute température on risque de sous produire beaucoup d'acétylène.

La demanderesse à découvert qu'il suffisait de porter le 1,2 dichloroéthane le plus vite possible à une température d'environ 500 à 750°C c'est à dire par mélange avec un fluide ou des particules très chaudes et de maintenir le 1,2 dichloroéthane par exemple pendant 0,010 à 0,25 s puis de tremper. On obtient ainsi une conversion élevée du 1,2 dichloroéthane et une très bonne sélectivité en chlorure de vinyle. La présente invention est donc un procédé de préparation du chlorure de vinyle par pyrolyse du 1,2 dichloroéthane qui consiste, en l'abscence de vapeur d'eau, à porter le 1,2-dichloroéthane à une température au moins égale à 480°C, en le mélangeant avec un flux de gaz chaud ou de particules chaudes, à maintenir ledit mélange à cette température pendant un temps compris entre 0,010 seconde et 0,5 seconde, à tremper le mélange, puis à séparer dudit mélange, le chlorure de vinyle formé.

La mélange peut s'effectuer par tout moyen, l'essentiel étant d'amener la chaleur le plus vite possible pour que le 1,2 dichloroéthane soit porté à une température telle qu'il se déshydrochlore en chlorure de vinyle et HCl. C'est très différent du procédé de l'art antérieur dans lequel le 1,2 dichloroéthane est chauffé dans les tubes d'un four. Le transfert de chaleur vers le 1,2 dichloroéthane liquide, ou gazeux si on l'a vaporisé auparavant, puis vers le 1,2 dichloroéthane gazeux pour le porter vers 400 ou 500°C et fournir l'énergie de deshydrochloration est lent puisque c'est un échange gaz/gaz : flamme ou fumées/paroi du tube/1,2 dichloroéthane gaz.

Il suffit selon l'invention de mélanger le courant contenant le 1,2 dichloroéthane avec soit un flux de gaz chaud tel que de l'azote, du méthane, du benzène, de l'éthylène, de l'HCl ou un flux de particules chaudes telles que de la silice, de l'alumine de type corindon, des silicoaluminates de l'attapulgite. Le mélange est instantané, la température du 1,2 dichloroéthane est instantanément élevée, il y a donc pyrolyse. La taille des particules peut être entre 10 et 500 microns de préférence entre 10 et 40 µm.

Les particules peuvent être transportées par un fluide (gaz de transport).

Le courant contenant le 1,2 dichloroéthane peut être du 1,2 dichloroéthane pur, impur ou contenant un produit qui ne gène pas la deshydrochloration (pyrolyse) du 1,2 dichloroéthane.

Il est plus simple d'utiliser un courant constitué seulement de 1,2 dichloroéthane.

La pyrolyse se déroule en l'absence d'eau.

Bien que, dès 400°C, on commence à pyrolyser le 1,2 dichloroéthane on opère au moins à 480°C et de préférence au-dessus de 550°C.

On préfère ne pas dépasser 800°C. Ceci est la température atteinte par le 1,2 dichloroéthane dès qu'il s'est mélangé avec le fluide ou le courant de particules à haute température. La demanderesse a trouvé qu'une température comprise entre 550 et 750°C et de préférence entre 550 et 650°C convenait.

Puis la température baisse par suite de la réaction endothermique de pyrolyse. On maintient le mélange constitué par le courant de 1,2 dichloroéthane (et ses produits de pyrolyse) et le fluide ou le courant de particules le temps nécessaire pour obtenir une conversion significative du 1,2 dichloroéthane. Cette durée est habituellement comprise entre 0,010 et 0,5 secondes et de préférence entre 0,050 et 0,200.

On se sortirait pas du cadre de l'invention en ajoutant dans le D12 ou dans le courant de particules des initiateurs de pyrolyse tels que par exemple du chlore ou des produits donnant du chlore tels que CCl₄, hexachloroéthane, chlorure de thionyle.

L'homme de l'art peut choisir la quantité de fluide ou de particules à haute température ainsi que cette température pour que la température reste suffisante pendant le temps de contact pour avoir une conversion significative.

La conversion augmente avec la température et le temps de contact.

Le mélange consitué par le courant contenant le 1,2 dichloroéthane avec le fluide ou le courant de particules à haute température peut se faire par un dispositif tel qu'un pulvérisateur ou deux tubes concentriques dont les sorties sont telles que les flux forment un angle pour mieux se mélanger. Ce peut être aussi des injecteurs dans un tube.

On obtient la fin de la réaction de pyrolyse en effectuant une trempe du milieu réactionnel c'est à dire de l'ensemble du courant contenant essentiellement le 1,2 dichloroéthane (et ses produits de pyrolyse) et du fluide ou du courant de particules à haute température. Cette trempe peut se faire avec du 1,2 dichloroéthane froid.

La réaction de pyrolyse se déroule par exemple dans un simple tuyau et on injecte en sortie du tuyau un flux de 1,2 dichloroéthane liquide froid puis l'ensemble passe dans un séparateur gaz liquide et ensuite on effectue les opérations conventionnelles de distillation pour récupérer le chlorure de vinyle, le 1,2 dichloroéthane, HCl, le fluide ou le gaz de transport et les sous produits de pyrolyse.

Si on a utilisé un courant de particules à haute température pour chauffer le 1,2 dichloroéthane on peut séparer les particules du milieu réactionnel par cyclonage ou tout système équivalent avant de faire la trempe.

### EXEMPLES 1 ET 2

On utilise un réacteur formé d'un tube horizontal en inconel. On injecte à l'une des extrémités à l'aide d'une buse le 1,2 dichloroéthane et de l'azote très chaud.

La buse est vendue sous le nom SONICORE ATOMIZERS, modèle 052 MB1 par SONIC Developpement Corporation - 305 Island Road - MAHWAH NEW JERSEY.

Elle est du type pulvérisateur, on injecte au centre dans l'axe le 1,2 dichloroéthane puis dans la chambre concentrique l'azote chaud. Pour éviter un bouchage par le coke coté 1,2 dichloroéthane on a introduit un manchon en céramique pour isoler thermiquement le 1,2 dichloroéthane jusqu'à son mélange avec l'azote à la sortie de la buse.

Le 1,2 dichloroéthane est envoyé dans le réacteur par l'intermédiaire d'une pompe péristaltique. La vitesse de rotation de la pompe est régulée et le poids de 1,2 dichloroéthane injecté est connu grâce à une balance. Ceci permet d'obtenir, par deux méthodes différentes, le débit de 1,2 dichloroéthane qui traverse le réacteur.

Dans cette buse, soudée à l'entrée du réacteur le 1,2 dichloroéthane, chauffé préalablement à 100 - 150°C vient se mélanger au fluide caloporteur (azote) qui lui fournit l'énergie nécessaire pour atteindre la température de réaction désirée.

En sortie de la buse, la réaction démarre dans le réacteur (tube en inconel de 12,5 mm de diamètre et de 825 mm de longueur) qui est isolé par des bandes chauffantes électriques ; l'ensemble étant noyé dans du calorifuge. Une série de thermocouples disposés axialement tout le long du réacteur nous informe en permanence sur le profil de température.

Selon la puissance des bandes chauffantes et la quantité de 1,2 dichloroéthane convertie on peut sur cet appareillage de petite taille fournir plus de chaleur que n'en consomme la pyrolyse endothermique.

En sortie du réacteur, deux jets d'azote à température ambiante refroidissent brutalement le mélange réactionnel pour l'amener en dessous de 400°C afin de stopper immédiatement la pyrolyse du 1,2 dichloroéthane.

Ces gaz sont ensuite mélangés avec du méthane (dont le débit est régulé) qui tient le rôle de traceur et permet de réaliser des bilans matières à partir des analyses chromatographiques.

Puis ils sont refroidis une deuxième fois par l'intermédiaire de deux échangeurs à eau jusqu'à la température ambiante. La fraction liquide est recueillie dans un ballon (condensation des hydrocarbures les plus lourds). La fraction gazeuse traverse dans un premier temps une colonne d'eau où est piégé le HCl, puis une colonne remplie de charbon actif où est piégé le VCM (chlorure de vinyle) puis est rejetée à l'atmosphère.

Les produits de la réaction sont analysés par chromatographie en phase gazeuse.

Toutes les informations données par es capteurs du pilote telles que le profil de température dans le réacteur, les débits d'azote et de 1,2 dichloroéthane, la pression dans le réacteur etc. sont enregistrées en permanence par un ordinateur. Ceci nous permet de connaître l'évolution de ces paramètres au cours du temps.

A la fin de chaque expérience le réacteur est purgé par de l'azote afin d'éviter la formation de mélange explosif.

Les résultats des exemples 1 et 2 figurent dans le tableau 1.

**T1** désigne la température atteinte par le 1,2 dichloroéthane dès qu'il s'est mélangé avec l'azote.

**T2** désigne la température moyenne du mélange réactionnel dans le réacteur.

La pression est en bars absolus.

La conversion désigne le pourcentage de 1,2 dichloroéthane qui a été pyrolysé.

La sélectivité en chlorure de vinyle indique le pourcentage du 1,2 dichloroéthane pyrolysé qui est devenu du chlorure de vinyle, et ainsi de suite pour les autres produits.

### EXEMPLE 3 (Comparatif)

On effectue la pyrolyse du 1,2 dichloroéthane dans un four de l'art antérieur (ULLMANN'S). Le 1,2 dichloroéthane est à 100°C à l'entrée du four puis il est chauffé, vaporisé, puis porté à 480°C. Les résultats sont reportés sur le Tableau 1.

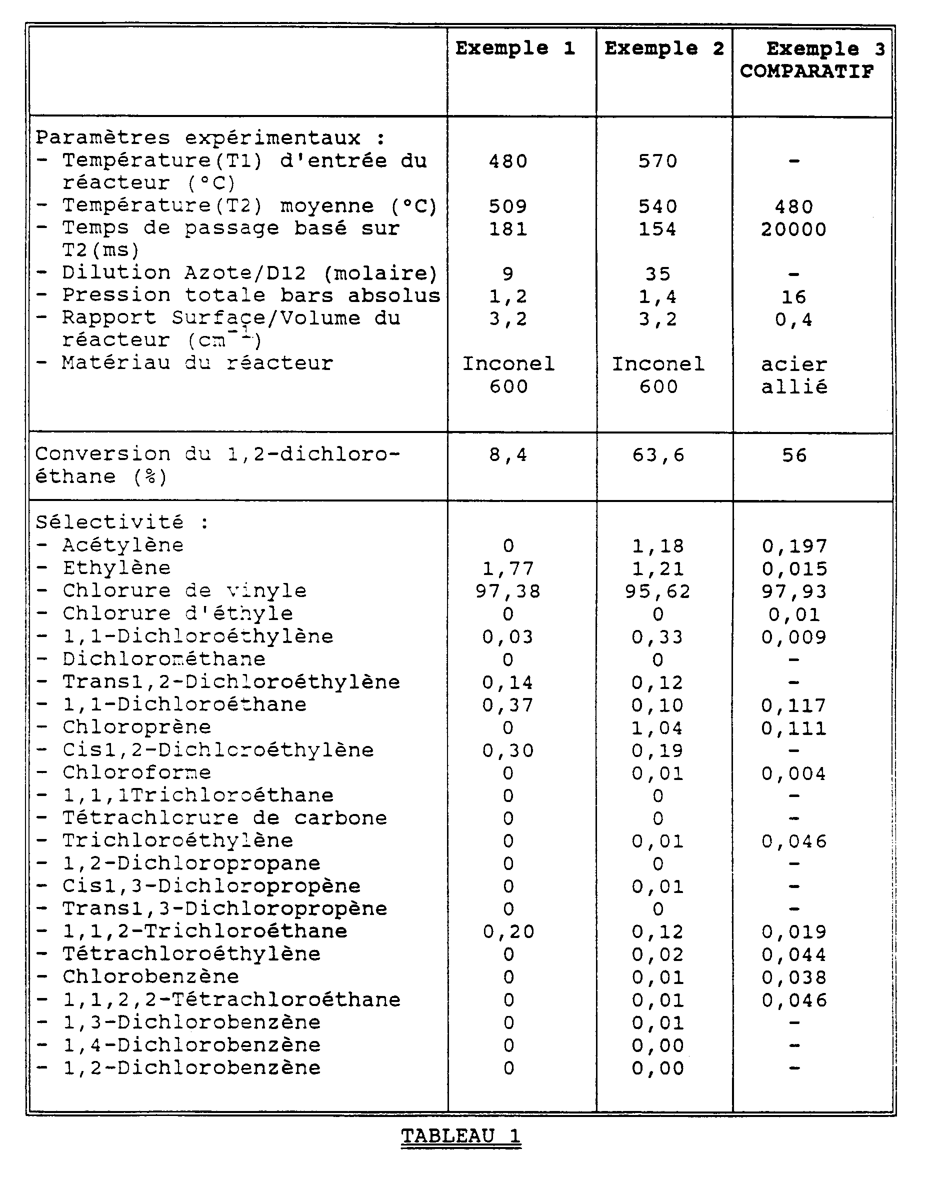

### EXEMPLES 4 et 5

On opère comme dans les exemples 1 et 2 mais avec des températures plus élevées et des temps de contact plus courts.

Les résultats sont sur le Tableau 2.

### EXEMPLE 6

On utilise le même tube d'inconel que dans les exemples précédents mais sans la buse de mélange et on le dispose verticalement. On dispose au dessus du tube un autre tube de diamètre supérieur relié au tube réacteur par un cône et servant à mélanger le sable et le 1,2 dichloroéthane. Au dessus de ce mélangeur on dispose un réservoir contenant du sable de silice de 100 microns chauffé à 1100°C.

On alimente le mélangeur par un mélange d'azote et de 1,2 dichloroéthane, on ajuste ce débit et éventuellement celui de sable pour avoir une température du gaz (c'est à dire du 1,2 dichloroéthane) à l'entrée du tube réacteur d'inconel entre 550 et 650°C.

Le temps de séjour du 1,2 dichloroéthane dans le mélangeur est négligeable devant le temps de séjour dans le tube en inconel.

En sortie du tube en inconel on dispose un réservoir cylindrique de 13 cm de diamètre et 26 cm de haut pour séparer le sable des gaz. La trempe se fait par un jet d'azote froid.

On a obtenu les mêmes résultats que dans l'exemple 5.

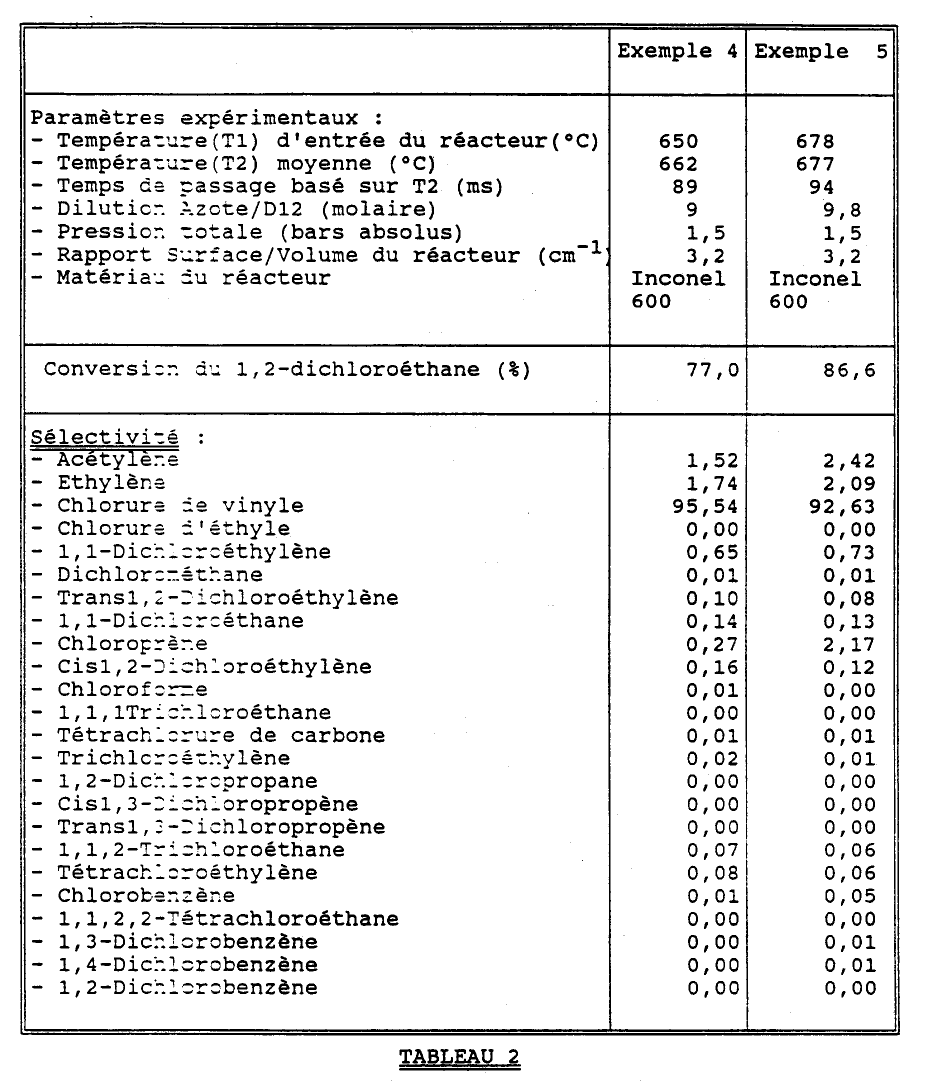

## Revendications

1. Procédé de préparation du chlorure de vinyle par pyrolyse du 1,2 dichloroéthane qui consiste, en l'abscence de vapeur d'eau, à porter le 1,2-dichloroéthane à une température au moins égale à 480°C, en le mélangéant avec un flux de gaz chaud ou de particules chaudes, à maintenir ledit mélange à cette température pendant un temps compris entre 0,010 seconde et 0,5 seconde, à tremper le mélange, puis à séparer dudit mélange le chlorure de vinyle forme.

2. Procédé selon la revendication 1, caractérisé en ce que le 1,2-dichloroéthane est porté au-dessus de 550°C.

3. Procédé selon la revendications 2 caractérisé en ce que le 1,2 dichloroéthane est porté entre 550°C et 650°C.

4. Procédé selon l'une des revendications 1 à 3 caractérisé en ce que le temps est compris entre 0,05 seconde et 0,2 seconde.

## Claims

1. Process for the preparation of vinyl chloride by pyrolysis of 1,2-dichloroethane which consists, in the absence of steam, in bringing the 1,2-dichloroethane to a temperature which is at least equal to 480°C, in mixing it with a flow of hot gas or or hot particles, in maintaining the said mixture at this temperature for a time of between 0.010 second and 0.5 second, in steeping the mixture and in then separating the vinyl chloride formed from the said mixture.

2. Process according to Claim 1, characterized in that the 1,2-dichloroethane is brought above 550°C.

3. Process according to claim 2, characterized in that the 1,2-dichloroethane is brought between 550°C and 650°C.

4. Process according to one of Claims 1 to 3, characterized in that the time is between 0.05 second and 0.2 second.

## Patentansprüche

1. Verfahren zur Herstellung von Vinylchlorid durch Pyrolyse von 1,2-Dichlorethan in Abwesenheit von Wasserdampf, das aus den folgenden Verfahrensschritten besteht: Erwärmen von 1,2-Dichlorethan bis zu einer Temperatur von mindestens 480 °C durch Vermischen mit einem Fluß eines heißen Gases oder heißer Teilchen, Belassen der Mischung bei dieser Temperatur für eine Zeit zwischen 0,010 Sekunden und 0,5 Sekunden, Abschrecken der Mischung, dann Abtrennen des gebildeten Vinylchlorids aus dieser Mischung.

2. Verfahren nach Anspruch 1, dadurch gekennzeichnet, daß das 1,2-Dichlorethan bis über 550 °C erwärmt wird.

3. Verfahren nach Anspruch 2, dadurch gekennzeichnet, daß das 1,2-Dichlorethan bis zu einer Temperatur zwischen 550 °C und 650 °C erwärmt wird.

4. Verfahren nach einem der Ansprüche 1 bis 3, dadurch gekennzeichnet, daß die Zeit 0,05 Sekunden bis 0,2 Sekunden beträgt.
